# EUROPÄISCHE PATENTANMELDUNG

(11) **EP 1 329 259 A2**
(43) Veröffentlichungstag der Anmeldung: **23.07.2003**
(21) Anmeldenummer: 03000648.0
(22) Anmeldetag: 16.01.2003
(51) Int. Cl.: B01J 19/24, C07B 41/06, C07B 41/02, C07B 37/04, C07B 37/00

(54) **Kontinuirliches Verfahren zur Hydrolyse einer lösungsmittelhaltigen organischen Verbindung**

(30) Priorität: 17.01.2002 DE 10201689
(71) Anmelder: MERCK PATENT GmbH, 64293 Darmstadt (DE)
(72) Erfinder: Grund, Michael, Dr., 64293 Darmstadt (DE); Stork, Holger, 64380 Rossdorf (DE); Riebel, Reinhold, 64354 Reinheim (DE); Schütz, Christoph, 64285 Darmstadt (DE)
(74) Vertreter: Isenbruck, Günter, Dr.

(57) **Zusammenfassung**

Es wird ein kontinuierliches Verfahren zur Hydrolyse einer lösungsmittelhaltigen organischen Verbindung oder eines lösungsmittelhaltigen Gemisches organischer Verbindungen vorgesehen, das man in einem Schlaufenreaktor mit einer äußeren Schlaufe, aufweisend ein Kreislaufrohr (1) und eine Umwälzpumpe (2) durchführt, wobei man einen Eduktstrom (A1) umfassend die lösungsmittelhaltige organische Verbindung oder das lösungsmittelhaltige Gemisch organischer Verbindungen und einen Eduktstrom (A2), enthaltend Wasser oder wässrige Salzsäurelösung auf der Druckseite der Pumpe (2) kontinuierlich zudosiert und das hydrolysierte Reaktionsgemisch (B) kontinuierlich aus dem Kreislaufrohr (1) abzieht.

## Beschreibung

Die Erfindung betrifft ein kontinuierliches Verfahren zur Hydrolyse einer lösungsmittelhaltigen organischen Verbindung oder eines lösungsmittelhaltigen Gemisches organischer Verbindungen.

Hydrolysereaktionen organischer Verbindungen sind häufig stark exotherm. Werden derartige Reaktionen in üblichen Rührkesselapparaturen mit Mantelkühlung durchgeführt, so kann die Reaktionswärme häufig nur ungenügend über das im Kühlmantel zirkulierende Wärmetauschmittel abgeführt werden. Es besteht die Gefahr, dass das Rührwerk keine ausreichende Durchmischung der Reaktionspartner sicherstellen kann, wodurch es zu lokalen Überhitzungen, sogenannten Hot-Spots kommt mit der Folge der Bildung von Nebenprodukten und insbesondere der lokalen Verdampfung von leichtsiedenden Komponenten, insbesondere des Lösungsmittels. Darüber hinaus verbleibt bei diskontinuierlichen, absatzweisen Verfahren in Rührkesseln bereits gebildetes Produkt über längere Zeit in der Reaktionsmischung und ist dort den Hot-Spots ausgesetzt, wodurch es zu Alterungsprozessen und der Bildung von Nebenprodukten kommt. Nachteilig sind auch die langen Belegungszeiten der Rührkessel, da zur Beherrschung der Hot-Spot-Problematik die Zudosierung der organischen Verbindung oder des Gemisches organischer Verbindungen zur vorgelegten Hydrolyselösung, in der Regel Wasser oder eine wässrige Mineralsäure, nur langsam erfolgen kann. Nachteilig ist auch, dass das bei der Hydrolyse im Rührkessel erhaltene Gemisch aus wässriger und das Wertprodukt umfassender organischer Phase in einem störungsanfälligen Arbeitsschritt vom Boden des Reaktors abgezogen werden muss, wobei sich eine trombische Strömung ausbilden kann, die zu verstärkter Vermischung von wässriger und organischer Phase führt. Der nachfolgende Arbeitsschritt, die Auftrennung von wässriger und organischer Phase, ist erschwert.

Demgegenüber war es Aufgabe der Erfindung, ein Verfahren zur Hydrolyse einer lösungsmittelhaltigen organischen Verbindung oder eines lösungsmittelhaltigen Gemisches organischer Verbindungen zu Verfügung zu stellen, das kontinuierlich durchführbar ist, und das sicherer und wirtschaftlicher durchführbar ist. Die Raumzeitausbeute und die Produktqualität sollen verbessert werden und nachfolgende Aufarbeitungsschritte mit geringerem Aufwand durchführbar werden.

Die Lösung geht aus von einem kontinuierlichen Verfahren zur Hydrolyse einer lösungsmittelhaltigen organischen Verbindung oder eines lösungsmittelhaltigen Gemisches organischer Verbindungen.

Die Erfindung ist dadurch gekennzeichnet, dass man das Verfahren in einem Schlaufenreaktor mit einer äußeren Schlaufe, aufweisend ein Kreislaufrohr und eine Umwälzpumpe durchführt, wobei man einen Eduktstrom umfassend die lösungsmittelhaltige organische Verbindung oder das lösungsmittelhaltige Gemisch organischer Verbindungen und einen Eduktstrom enthaltend Wasser oder wässrige Salzsäurelösung auf der Druckseite der Pumpe kontinuierlich zudosiert und das hydrolysierte Reaktionsgemisch kontinuierlich aus dem Kreislaufrohr abzieht.

Schlaufenreaktoren sind in der chemischen Verfahrenstechnik bekannt und beispielsweise im Lehrbuch der technischen Chemie von M. Baerns: Chemische Reaktionstechnik, 2. Auflage 1992, Verlag Thieme, beschrieben. In derartigen Reaktoren wird das Reaktionsgemisch kontinuierlich mit hoher Turbulenz umgewälzt, und kontinuierlich ein gegenüber dem umgewälzten Strom wesentlich geringerer Eduktstrom von außen zugeführt bzw. ein entsprechender Produktstrom aus dem Reaktor abgezogen.

Der Schlaufenreaktor kann als zylindrischer Apparat mit zentralem Leitrohr ausgebildet sein, wobei die Umwälzung zwischen Leitrohr und dem Zwischenraum zwischen Leitrohr und Reaktorinnenwand in einer inneren Schlaufe erfolgt oder in Form eines Kreislaufrohrs, durch das die Umwälzung mittels einer Pumpe bewirkt wird (Schlaufenreaktoren mit sogenannter äußerer Schlaufe). Die vorliegende Erfindung betrifft einen Schlaufenreaktor mit äußerer Schlaufe. Derselbe umfasst als Hauptelemente ein Kreislaufrohr und eine Umwälzpumpe. Bei bekannten Schlaufenreaktoren mit äußerer Schlaufe, wie sie beispielsweise in der oben zitierten Literaturstelle beschrieben sind, wird ein Eduktstrom, der im Vergleich zum umgewälzten Flüssigkeitsstrom gering ist, auf der Saugseite der Pumpe zugeführt und ein entsprechender Produktstrom auf der Druckseite der Pumpe abgezogen. Die Pumpe bewirkt eine hohe Strömungsgeschwindigkeit im Umlauf und damit eine turbulente Strömung, die für eine gute Durchmischung im Kreislaufrohr sorgt.

Erfindungsgemäß wird entgegen der in Fachkreisen üblichen Regel, nicht gegen den Druck der Pumpe zu dosieren, der Eduktstrom aus der lösungsmittelhaltigen organischen Verbindung oder dem lösungsmittelhaltigen Gemisch organischer Verbindungen und Wasser auf der Druckseite der Pumpe kontinuierlich zudosiert. In überraschender Weise kann dadurch die Hot-Spot-Problematik besonders gut beherrscht werden.

Die bei der Hydrolyse freiwerdende Wärme wird zunächst kapazitiv, das heißt durch einen Temperaturanstieg im Umlaufstrom aufgenommen. Häufig wird sie anschließend über einen oder mehrere im Kreislaufrohr angeordnete Wärmetauscher, entsprechend der im Einzelfall durchgeführten Hydrolysereaktion und der entwickelten Wärmemenge, abgeführt. Es ist jedoch auch möglich, die freiwerdende Wärme mit dem aus dem Kreislaufrohr abgezogenen Produktstrom abzuführen, sofern die Temperaturerhöhung nicht in kritische Bereiche führt, wie zum Sieden bzw. zur Produktschädigung. Dies ist beispielsweise möglich bei Reaktionen mit geringer Exothermie oder bei sehr kalten Edukten aus Tiefkalt-Reaktionen.

Bevorzugt werden im Kreislaufrohr nach der Stelle, an der das zu hydrolysierende Reaktionsgemisch zudosiert wird, ein oder mehrere statische Mischer angeordnet. Dadurch wird die Durchmischung des Reaktionsgemisches verbessert, mit der Folge einer besseren Gleichverteilung der Wärme und weiteren Reduzierung der Hot-Spot-Problematik.

In einer bevorzugten Ausführungsform ist im Kreislaufrohr ein Pufferbehälter für das Reaktionsgemisch auf der Saugseite der Pumpe mit einem Überlauf für das abzuziehende hydrolysierte Reaktionsgemisch angeordnet.

Das Volumen des Pufferbehälters wird in vorteilhafter Weise so gewählt, dass die Verweilzeit des hydrolysierten Reaktionsgemisches im Pufferbehälter 10 Minuten, bevorzugt 5 Minuten, besonders bevorzugt 1 Minute, nicht übersteigt.

Um Dosierschwankungen auszugleichen sowie um Luft, insbesondere Gas aus dem System zu entfernen wäre es vorteilhaft, das Volumen des Pufferbehälters möglichst groß auszulegen. Das Volumen des Pufferbehälters ist jedoch in vorteilhafter Weise, wie vorstehend definiert, nach oben zu begrenzen, damit der Schlaufenreaktor möglichst schnell in den stationären Betriebszustand übergeht, sowie aus Sicherheitsgründen.

Aus dem Produkt der vorgegebenen Verweilzeit und dem Volumenstrom im Kreislaufrohr lässt sich das ungefähre Volumen des Pufferbehälters errechnen.

Bevorzugt beträgt das Verhältnis zwischen dem Volumenstrom im Kreislaufrohr und dem zudosierten Volumenstrom 5 : 1 bis 15 : 1, insbesondere 10 : 1.

Das aus dem Kreislaufrohr, bevorzugt als Überlauf des Pufferbehälters abgezogene hydrolysierte Reaktionsgemisch wird vorzugsweise kontinuierlich einem Phasenscheider zugeführt. Im Phasenscheider wird die das Wertprodukt umfassende organische Phase, die häufig die Oberphase ist, von der wässrigen Phase, häufig die Unterphase, abgetrennt.

In einer bevorzugten Ausführungsvariante ist die organische Verbindung der Anlagerungskomplex von Aluminiumtrichlorid an die Carbonylgruppe des Acylierungsproduktes einer Friedel-Crafts-Reaktion, wobei die Hydrolyse in wässriger Salzsäure durchgeführt wird.

Friedel-Crafts-Acylierungen aromatischer Verbindungen sind als wichtigste Synthesemethode für aromatische und aromatisch/aliphatische Ketone bekannt. Die aromatische Verbindung wird mit einem Acylierungsmittel, in der Regel einem Säurehalogenid, häufig einem Säurechlorid, einem Säureanhydrid oder gegebenenfalls einer Carbonsäure in Gegenwart von Aluminiumtrichlorid als Fiedel-Crafts-Katalysator umgesetzt. Dabei bildet sich zunächst der Anlagerungskomplex des Aluminiumtrichlorids an die Carbonylgruppe des Acylierungsprodukts, der anschließend hydrolysiert wird, häufig in wässriger Salzsäurelösung, und schließlich extrahiert wird.

Bezüglich der einsetzbaren Aromaten gibt es keine grundsätzlichen Einschränkungen; eingesetzt werden können monocyclische oder polycyclische, unsubstituierte oder substituierte Aromaten, weiterhin Heterocyclen. In jedem Fall muss nach Beendigung der Reaktion der gebildete Komplex aus Keton und Aluminiumchlorid hydrolytisch gespalten werden. Diese hydrolytische Spaltung wird erfindungsgemäß in einem Schlaufenreaktor mit äußerer Schlaufe durchgeführt.

Bevorzugt ist der nach Friedel-Crafts acylierte Aromat trans-n-Alkyl-cyclohexyl-benzol, wobei der n-Alkylrest bevorzugt 2 bis 7 Kohlenstoffatome, besonders bevorzugt 2, 3 oder 5 Kohlenstoffatome, enthält.

Bevorzugt werden als Lösungsmittel eine oder mehrere der Verbindungen Toluol, Benzol, Xylol, Oktan, Hexan, Heptan, Tetrahydrofuran, Dichlormethan, Chloroform oder Diethylenglykol eingesetzt. Im erfindungsgemäßen Verfahren können auch niedrigsiedende Lösungsmittel, wie Dichlormethan oder Chloroform problemlos in dem stark exothermen Hydrolyseverfahren eingesetzt werden, da die aus Rührwerksapparaturen bekannte Hot-Spot-Problematik im erfindungsgemäßen Verfahren nicht auftritt.

In einer bevorzugten Verfahrensvariante ist die organische Verbindung das Magnesium- oder Lithiumalkoholat, das durch Addition eines Ketons an eine metallorganische Verbindung gebildet wurde.

In einer weiteren Verfahrensvariante ist die organische Verbindung der Palladiumanlagerungskomplex, der in einer Suzuki-Kopplung durch Reaktion einer Arylboronsäure mit einem Arylbromid gebildet wurde.

Gemäß einer anderen Verfahrensvariante ist die organische Verbindung ein Alkoholat, das durch Koordination des Aluminiumatoms von Lithiumaluminiumhydrid am Sauerstoffatom der Carbonylgruppe einer organischen Säure gebildet wurde.

In einer weiteren Alternative ist die organische Verbindung ein Borsäuredimethylester, der durch Umsetzung einer bifluorierten organischen Verbindung mit Lithiumdiisopropylamin und Trimethylborat erhalten wurde.

Die Erfindung umfasst auch ein Verfahren, wonach die organische Verbindung ein Oxaphosphetan ist, das durch Wittig-Reaktion eines Aldehyds oder Ketons mit einem Phosphoniumjodid erhalten wurde.

Nach dem erfindungsgemäßen Verfahren werden bevorzugt Verbindungen erhalten, die als Zwischenprodukte zur Herstellung von Flüssigkristallen dienen.

Das erfindungsgemäße Verfahren hat somit den Vorteil, dass stark exotherme Hydrolysereaktionen lösungsmittelhaltiger organischer Verbindungen oder Gemische organischer Verbindungen sicher beherrschbar sind, da der reaktionsfähige Eduktstrom zu einem wesentlich größeren Kreislaufstrom aus bereits abreagiertem und somit quasi inertem Reaktionsgemisch zudosiert wird, der den Eduktstrom verdünnt. Somit ist die Temperaturerhöhung pro Volumenelement durch das Verhältnis von Edukt- zu Kreislaufstrom exakt kontrollier- und einstellbar. Die Temperaturschwankungen können auf einen engen Bereich, von etwa ± 3°C, begrenzt werden. Dadurch kann das Sieden, insbesondere des Lösungsmittels, und somit die Hot-Spot-Problematik vermieden werden. Auch bei Überdosierung eines Reaktanten ist stets ausreichend kapazitive Kühlleistung vorhanden, da die Stoffmenge der Reaktanten gegenüber dem Kreislaufstrom klein ist. Wird ein Reaktand zu stark überdosiert, so reichert er sich lediglich im System an und die Reaktion und somit die freiwerdende Reaktionsenergie kann weiterhin über die Dosierung des zweiten Reaktanden gesteuert werden.

Durch einfache Temperaturmessungen, über die die Dosierrate gesteuert wird, ist der Schlaufenreaktor leicht regelbar auszuführen. Bei einer Störung, beispielsweise Überdosierung eines Reaktanden stellt sich von selbst ein neuer stabiler, stationärer Betriebspunkt ein. Dieses rein proportionale Verhalten des Systems ist sehr leicht automatisierbar.

Zusätzlich zu den kleinen Mengen der Reaktanden im System ist auch das eigentliche Gesamtvolumen im Schlaufenreaktor sehr klein, in der Regel kleiner als 10 %, häufig < als 5 % eines Rührkessels mit gleicher Raumzeitausbeute, so dass das Gefahrenpotential wesentlich geringer ist. Durch das kleine Reaktionsvolumen im Schlaufenreaktor ist die Verweilzeit sehr klein, so dass Alterung des Materials und weitere zeitabhängige Prozesse kaum eine Rolle spielen. Des weiteren ist eine kontinuierliche Aufarbeitung des hydrolysierten Reaktionsgemisches in einem nachfolgenden Extraktionsschritt möglich. Dies führt zu einer besseren Auslastung der vorhandenen Reaktoren zur Herstellung der zu hydrolysierenden organischen Verbindungen, da das zusätzliche Volumen des Extraktionsmittels sowie der Hydrolyselösung nicht bei der Apparateauslegung berücksichtigt werden muss. Somit können entweder kleinere Apparate zur Synthese vorgesehen werden oder es kann die Ansatzgröße bei vorhandenen Apparaten vergrößert und gleichzeitig, bei entsprechender Auslegung, die Zykluszeit verkürzt werden.

Durch die hohe Strömungsgeschwindigkeit im Kreislaufstrom wird eine turbulente Strömung aufrechterhalten, die für eine gute Durchmischung der bei der Hydrolyse freiwerdenden Energie, gleichmäßig über den ganzen Reaktorquerschnitt sorgt, wodurch Hot-Spots sicher vermieden werden. Durch die feine Dispersion der Phasen entsteht eine große Phasengrenzfläche, die den Stoffaustausch stark beschleunigt. Die bei der Hydrolyse entstehenden Salze gehen somit sehr viel leichter und vollständiger in die wässrige Phase über, mit der Folge, dass die Aufarbeitung in wenigen Extraktionsschritten möglich ist, was bezüglich Wasserverbrauch und Umweltschutz ein weiterer Vorteil ist.

Die modulare Bauweise des Hydrolysereaktors ermöglicht ein kompaktes Design, das den Platzbedarf klein hält. Darüber hinaus kann das Modul transportabel ausgeführt werden, so dass es an verschiedenen Stellen im Betrieb eingesetzt werden kann. Dies erfordert eine robuste Ausführung, die dann auch imstande ist, eventuell auftretende Feststoffausfälle im Kreislauf problemlos zu verarbeiten.

Nach dem erfindungsgemäßen Verfahren lässt sich somit das Gefahrenpotential von Hydrolysereaktoren drastisch reduzieren. Die Raumzeitausbeute wird durch bessere Reaktorauslastung und kürzere Belegungszeiten erhöht, die Produktqualität verbessert und nachfolgende Aufarbeitungsschritte sind mit geringerem Aufwand verbunden.

Die Erfindung wird im Folgenden anhand einer Zeichnung und eines Ausführungsbeispiels näher erläutert.

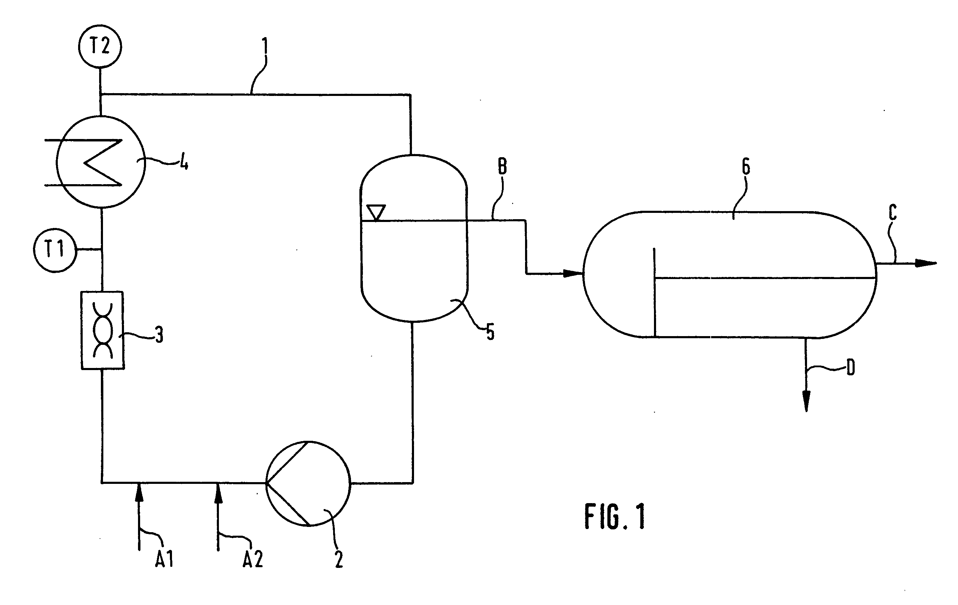

- Figur 1: zeigt die schematische Darstellung einer bevorzugten Anlage zur Durchführung des erfindungsgemäßen Verfahrens.

Hierbei wird ein Schlaufenreaktor mit äußerer Schlaufe eingesetzt, umfassend ein Kreislaufrohr 1 und eine Pumpe 2. Die Eduktströme wässrige Salzsäurelösung, A1 und organische Verbindung, A2, werden auf der Druckseite der Pumpe 2 zudosiert und auf der Saugseite der Pumpe wird ein Produktstrom B aus hydrolysiertem Reaktionsgemisch abgezogen.

In der in Fig. 1 dargestellten besonderen Ausführungsvariante ist im Kreislaufrohr 1 nach der Zudosierung der Eduktströme A1 und A2 ein Statikmischer 3 und stromabwärts desselben ein Wärmetauscher 4 angeordnet. Vor und nach dem Wärmetauscher 4 sind Temperaturmessstellen T1 bzw. T2 angeordnet. Auf der Saugseite der Pumpe 2 ist im Kreislaufrohr 1 ein Pufferbehälter 5 vorgesehen, dessen Überlauf B den Produktstrom bildet. Der aus dem Kreislaufrohr abgezogene Produktstrom B wird einem Phasenscheider 6 zugeführt und dort in eine organische Oberphase C und eine wässrige Unterphase D aufgetrennt.

### Beispiel

In einer Anlage entsprechend der schematischen Darstellung in Fig. 1 wurde in ein Kreislaufrohr mit einem Innendurchmesser von 25 mm kontinuierlich ein Eduktstrom umfassend den Anlagerungskomplex von Aluminiumchlorid an das Acetylierungsprodukt von trans-n-Propyl-Cyclohexyl-Benzol als 10%ige Lösung in Dichlormethan mit einem Volumenstrom von 175 I/h zudosiert. Gleichzeitig wurde ein Volumenstrom von 90 I/h 5 %ige wässrige Salzsäurelösung zudosiert.

Stromabwärts der Dosierstellen für die Reaktionslösung und die wässrige Salzsäure war ein statischer Mischer SMX® der Fa. Sulzer AG, Winterthur, angeordnet. Die Reaktionsmischung wurde anschließend einem mit einer Solelösung von -10°C gekühlten Wärmetauscher mit einer Leistung von ca. 7 KW/h zugeführt, wodurch die Temperatur der Reaktionslösung von 25°C vor dem Wärmetauscher auf 16°C nach dem Wärmetauscher abgesenkt wurde. Der durch das Kreislaufrohr mit einer Geschwindigkeit von ca. 1 m/s geführte Umlaufstrom betrug ca. 1700 I/h. Aus dem auf der Saugseite der Pumpe angeordneten Pufferbehälter wurde kontinuierlich als Überlauf ein Produktstrom abgezogen und in einem Phasenscheider in eine wässrige Oberphase von ca. 90 I/h und eine organische Unterphase von ca. 175 l/h aufgetrennt. Der Betrieb der Anlage funktionierte störungsfrei.

Demgegenüber erscheint die Zudosierung der Edukte auf der Saugseite der Kreislaufpumpe zunächst als vorteilhaft, da die Dosierpumpen in diesem Fall nicht gegen den Druck der Kreislaufpumpe arbeiten müssen.

Allerdings begünstigt das niedrige Druckniveau auf der Saugseite der Kreislaufpumpe die partielle Verdampfung des Lösungsmittels, insbesondere sofern es sich um niedrigsiedendes Lösungsmittel handelt, wenn man sich dem Siedepunkt des Lösungsmittels nähert. Die entstehenden Dampfblasen auf der Saugseite und in der Kreislaufpumpe führen zu Kavitation und somit zur Beschädigung der Kreislaufpumpe sowie zu einem starken Einbruch der Förderleistung. Dadurch vermindert sich auch die Kühlleistung, was zur weiterer Verdampfung führt. Dies hat den Zusammenbruch des Schlaufenstromes zur Folge, wenn beispielsweise durch Dosierfehler in kritische Temperaturbereiche vorgestoßen wird.

Das erfindungsgemäße Verfahren mit Dosierung auf der Druckseite der Kreislaufpumpe ist dagegen wesentlich stabiler und einfacher zu regeln, da eventuell entstehende Dampfblasen bereits im Wärmetauscher kollabieren, bevor sie die Kreislaufpumpe passieren. So kommt es nicht zum Zusammenbruch des Schlaufenstromes. Darüber hinaus ist das kritische Temperaturniveau auf der Druckseite der Kreislaufpumpe erhöht und somit die Problematik in Verbindung mit der Entstehung von Dampfblasen bereits dadurch entschärft.

## Patentansprüche

1. Kontinuierliches Verfahren zur Hydrolyse einer lösungsmittelhaltigen organischen Verbindung oder eines lösungsmittelhaltigen Gemisches organischer Verbindungen, **dadurch gekennzeichnet, dass** man das Verfahren in einem Schlaufenreaktor mit einer äußeren Schlaufe, aufweisend ein Kreislaufrohr (1) und eine Umwälzpumpe (2) durchführt, wobei man einen Eduktstrom (A1) umfassend die lösungsmittelhaltige organische Verbindung oder das lösungsmittelhaltige Gemisch organischer Verbindungen und einem Eduktstrom (A2) enthaltend Wasser oder wässrige Salzsäurelösung auf der Druckseite der Pumpe (2) kontinuierlich zudosiert und das hydrolysierte Reaktionsgemisch (B) kontinuierlich aus dem Kreislaufrohr (1) abzieht.

2. Verfahren nach Anspruch 1, **dadurch gekennzeichnet, dass** das Verhältnis aus dem Volumenstrom im Kreislaufrohr (1) und dem zudosierten Volumenstrom im Bereich von 5 : 1 bis 15 : 1, bevorzugt bei 10 : 1 liegt.

3. Verfahren nach Anspruch 1 oder 2, **dadurch gekennzeichnet, dass** im Kreislaufrohr (1) nach der Dosierstelle für die Eduktströme (A1, A2) ein oder mehrere Wärmetauscher (4) angeordnet sind.

4. Verfahren nach einem der Ansprüche 1 bis 3, **dadurch gekennzeichnet, dass** nach der Dosierstelle für die Eduktströme (A1, A2) und vor dem Wärmetauscher (den Wärmetauschern) (4) ein oder mehrere statische Mischer (3) angeordnet sind.

5. Verfahren nach einem der Ansprüche 1 bis 4, **dadurch gekennzeichnet, dass** im Kreislaufrohr (1) auf der Saugseite der Pumpe (2) ein Pufferbehälter (5) mit einem Überlauf vorgesehen ist, aus dem kontinuierlich das hydrolysierte Reaktionsgemisch (B) abgezogen wird.

6. Verfahren nach Anspruch 5, **dadurch gekennzeichnet, dass** man das Volumen des Pufferbehälters (5) dergestalt dimensioniert, dass die Verweilzeit des hydrolysierten Reaktionsgemisches (B) im Pufferbehälter (5) maximal 10 Minuten, bevorzugt maximal 5 Minuten, bevorzugt maximal 1 Minute, beträgt.

7. Verfahren nach einem der Ansprüche 1 bis 6, **dadurch gekennzeichnet, dass** das aus dem Kreislaufrohr (1)abgezogene hydrolysierte Reaktionsgemisch (B) kontinuierlich einem Phasenscheider (6) zugeführt wird.

8. Verfahren nach einem der Ansprüche 1 bis 7, **dadurch gekennzeichnet, dass** die organische Verbindung der Anlagerungskomplex von Aluminiumtrichlorid an die Carbonylgruppe des Acylierungsproduktes einer Friedel-Crafts-Reaktion ist und dass die Hydrolyse in wässriger Salzsäurelösung durchgeführt wird.

9. Verfahren nach Anspruch 8, **dadurch gekennzeichnet, dass** in der Friedel-Crafts-Reaktion ein trans-n-Alkyl-cylcohexyl-benzol acetyliert wird, wobei der n-Alkyl-Rest insbesondere 2 bis 7 Kohlenstoffatome, besonders bevorzugt 2, 3 oder 5 Kohlenstoffatome, enthält.

10. Verfahren nach Anspruch 8 oder 9, **dadurch gekennzeichnet, dass** das Lösungsmittel eine oder mehrere der Verbindungen Toluol, Benzol, Xylol, Oktan, Hexan, Heptan, Tetrahydrofuran, Dichlormethan, Chloroform oder Diethylenglykol ist.

11. Verfahren nach einem der Ansprüche 1 bis 7, **dadurch gekennzeichnet, dass** die organische Verbindung das Magnesium- oder Lithiumalkoholat ist, das durch Addition eines Ketons an eine metallorganische Verbindung gebildet wurde.

12. Verfahren nach einem der Ansprüche 1 bis 7, **dadurch gekennzeichnet, dass** die organische Verbindung der Palladiumanlagerungskomplex ist, der in einer Suzuki-Kopplung durch Reaktion einer Arylboronsäure mit einem Arylbromid gebildet wurde.

13. Verfahren nach einem der Ansprüche 1 bis 7, **dadurch gekennzeichnet, dass** die organische Verbindung ein Alkoholat ist, das durch Koordination des Aluminiumatoms von Lithiumaluminiumhydrid am Sauerstoffatom der Carbonylgruppe einer organischen Säure gebildet wurde.

14. Verfahren nach einem der Ansprüche 1 bis 7, **dadurch gekennzeichnet, dass** die organische Verbindung ein Borsäuredimethylester ist, der durch Umsetzung einer bifluorierten organischen Verbindung mit Lithiumdiisopropylamin und Trimethylborat erhalten wurde.

15. Verfahren nach einem der Ansprüche 1 bis 7, **dadurch gekennzeichnet, dass** die organische Verbindung ein Oxaphosphetan ist, das durch Wittig-Reaktion eines Aldehyds oder Ketons mit einem Phosphoniumjodid erhalten wurde.
